# EUROPÄISCHE PATENTSCHRIFT

(11) **EP 3 562 540 B1**
(45) Veröffentlichungstag und Bekanntmachung des Hinweises auf die Patenterteilung: **17.04.2024**
(21) Anmeldenummer: 18700448.6
(22) Anmeldetag: 02.01.2018
(51) Int. Cl.: A61M 39/12, B21D 39/04

(54) **VERFAHREN UND VORRICHTUNG ZUM AUFSTECKEN EINES SCHLAUCHES AUF EINE FORMSTABILE TÜLLE**
METHOD AND DEVICE FOR FITTING A HOSE ONTO A DIMENSIONALLY STABLE CONNECTOR
PROCÉDÉ ET DISPOSITIF POUR EMMANCHER UN FLEXIBLE SUR UNE TÉTINE D'ÉTANCHÉITÉ DE FORME STABLE

(30) Priorität: 02.01.2017 DE 102017000008
(43) Veröffentlichungstag der Anmeldung: 06.11.2019
(73) Patentinhaber: Fresenius Medical Care Deutschland GmbH, 61352 Bad Homburg (DE)
(72) Erfinder: GLASER, Benedict, 97421 Schweinfurt (DE)
(74) Vertreter: Herrmann, Uwe
(86) Internationale Anmeldenummer: PCT/EP2018/050043
(87) Internationale Veröffentlichungsnummer: WO 2018/122404

(56) Entgegenhaltungen:
- GB-A- 854 724
- JP-A- 2004 106 151
- US-A- 1 322 843
- US-A- 5 143 573
- US-A- 5 718 032
- "Wie bringe ich einen 9.8mm Schlauch auf einen 10mm Metalldorn", , 13. Mai 2016 (2016-05-13), XP055462117, Gefunden im Internet: URL:https://www.1-2-do.com/de/projekt/Wie- bringe-ich-einen-9_-3-_8mm-Schlauch-auf-ei nen-10mm-Metalldorn_-2-_/bauanleitung-zum- selber-bauen/4005163/ [gefunden am 2018-03-22]

## Beschreibung

Die vorliegende Erfindung betrifft ein Verfahren und eine Vorrichtung zum Aufstecken eines elastischen Schlauches auf eine formstabile Tülle.

Aus dem Stand der Technik ist es bekannt, zur Herstellung von Fluidverbindungen Schläuche per Hand auf Komponenten von Dialysegeräten bzw. auf an den Komponenten befindliche Tüllen aufzustecken. Bei den Komponenten kann es sich beispielsweise um Kammern, Sensoren, Aktoren, hydraulische Verbinder etc. handeln.

Ein Problem bei diesem Vorgang besteht darin, dass ein elastischer Schlauch, d.h. ein nicht vorstabiler Gegenstand, wie z.B. ein Silikonschlauch auf eine formstabile Tülle gesteckt werden muss, ohne dass der Schlauch unzulässig deformiert wird. Derzeit wird der Vorgang per Hand ausgeführt und das Ergebnis des Aufsteckens optisch kontrolliert.

Anzustreben ist ein automatisiertes Aufstecken der Schläuche, weil dadurch der aufwändige händische Montagevorgang ersetzt werden kann. Grundsätzlich ist es möglich, aufgrund des fortgeschrittenen heutigen Standes der Automatisierungstechnik bzw. Robotik die heute von Hand vorgenommenen Vorgänge automatisiert durchführen zu lassen. Jedoch besteht auch dabei das genannte Problem, dass sich nicht formstabile Schläuche nicht ohne weiteres auf eine formstabile Tülle aufstecken lassen, ohne dass es zu einer Verformung des Schlauches und somit zu einer Undichtigkeit eines Hydrauliksystems etc. kommen kann.

Eine denkbare Lösung dieses Problems besteht in der Verwendung von formstabilen Endstücken bzw. von Konnektoren, die an den Schläuchen montiert werden. Dies ist jedoch mit einem zusätzlichen Arbeitsschritt und mit Kostennachteilen verbunden.

Aus der US 5,718,032 ist es bekannt, einen Schlauch auf eine Tülle zu stecken, wobei der Schlauch mittels eines Schlauchmontagewerkzeuges gehalten wird und der aus diesem überstehende Schlauchabschnitt durch Druckluft aufgeweitet wird, die aus der Tülle ausströmt.

Die US 5,143,573 offenbart, ein flexibles Materialelement vorzusehen, das einen rohrförmigen Körper aufweist. Der rohrförmige flexible Körper wird zunächst über ein Stützrohr mit einem Stützrohrdurchmesser geschoben. Das Stützrohr, das den flexiblen Körper trägt, ist axial über ein Übertragungsstück mit einem Tragrohr verbunden. Einem Austrittsbereich des Übergangsstücks wird Druckluft zugeführt und der schlauchförmige flexible Körper wird auf einem Luftpolster, das durch die an der Austrittszone austretende Druckluft aufgebaut wird, vom Stützrohr über das Übergangsstück vollständig über das Tragrohr geschoben.

Aus der US 1,322,843 ist es ein Verfahren zum Einsetzen und Entformen von Kunststoffartikeln und -gegenständen bekannt, das darin besteht das darin besteht, den Gegenstand auf einen Former zu legen, dann die Endbearbeitung des Gegenstands vorzunehmen, dann ein Fluid unter Druck zwischen den Former und den fertigen Gegenstand einzuführen und dann den fertigen Gegenstand von dem Former zu entfernen.

Die GB 854 724 offenbart eine Streckschürze für eine Textilmaschine, die aus inneren und äußeren Schichten aus Gummi, gummiähnlichen Polymeren wie Butadien-Acrylnitril-Copolymeren oder weichgemachtem Polyvinylchlorid besteht.

Die JP 2004 106151 A offenbart ein Verfahren und eine Vorrichtung zum Anbringen und Abnehmen von Zubehörteilen wie Schläuchen und dergleichen, die an einen Flüssigkeitsauslass eines Sprühgeräts angeschlossen werden.

Der vorliegenden Erfindung liegt daher die Aufgabe zugrunde, ein Verfahren der eingangs genannten Art dahingehend weiterzubilden, dass ein Aufstecken eines Schlauches auf eine Tülle zuverlässig so vorgenommen werden kann, dass keine Verformung des Schlauches stattfindet.

Die Aufgabe wird durch eine Vorrichtung mit den Merkmalen des Anspruchs 1 gelöst.

Erfindungsgemäßist vorgesehen, dass das Verfahren mittels der erfindungsgemäßen Vorrichtung die folgenden Schritte umfasst, die in der Reihenfolge a) bis e) oder auch in einer davon abweichenden Reihenfolge durchgeführt werden können.
a) Erzeugen einer Strömung von unter Druck stehendem Gas aus der Tülle,
b) Heranführen des Schlauchendes an die Tülle entgegen der Strömungsrich tung des Gases aus der Tülle,
c) Verringern des Strömungsquerschnitts des Schlauchs hinter der Konnektionsstelle zur Erzeugung eines das Schlauchende erweiternden Staudrucks im Schlauch mittels eines Schlauchmontagewerkzeuges, mittels dessen der Schlauch abgesperrt oder dessen Durchfluss gedros-selt wird,
d) Aufstecken des Schlauchendes auf die Tülle und
e) Vergrößern des Strömungsquerschnitts des Schlauchs hinter der Konnektionsstelle zum Anlegen des Schlauchendes an die Außenseite der Tülle, wobeider Schlauch mittels des Schlauchmontagewerkzeugs gehalten und auf die Tülle aufgesteckt wird und vorgesehen ist, dass die Verringerung des Strömungsquerschnitts gemäß Schritt c) sowie die Vergrößerung des Strömungsquerschnitts gemäß Schritt e) mittels des Schlauchmontagewerkzeugs vorgenommen werden, wobei die Vergröße-rung des Strömungsquerschnitts gemäß Schritt e) durch Freigeben des Schlauches aus dem Schlauchmontagewerkzeug erfolgt.

Durch das Verringern des Strömungsquerschnitts des Schlauchs hinter (d.h. in Strömungsrichtung der Gasströmung stromabwärts der Konnektionsstelle zwischen dem Schlauchende und der Tülle) entsteht in dem Schlauchende, das auf die Tülle aufzustecken ist, ein das Schlauchende im Durchmesser erweiternder Staudruck. Diese Durchmesservergrößerung erlaubt es, das Schlauchende über die Tülle zu schieben, vorzugsweise ohne dass das Schlauchende die Tülle berührt. Die Konnektionsstelle bezeichnet die Längsposition am Schlauch, an der das Ende der aufgesteckten Tülle liegt.

Hat das Schlauchende relativ zu der Tülle seine gewünschte Position erreicht, wird der Staudruck im Schlauch verringert bzw. aufgehoben, so dass sich das Schlauchende an die Tülle anlegt. Dies kann beispielsweise dadurch erfolgen, dass der Strömungsquerschnitt des Schlauchs hinter der Konnektionsstelle vergrößert wird und/oder dadurch, dass die Gasströmung verringert oder ganz abgestellt wird.

Es wird darauf hingewiesen, dass der Begriff "Schlauchende" nicht das eigentliche stirnseitige Ende des Schlauches umfasst, sondern einen Bereich mit einer gewissen Länge, der das eigentliche Schlauchende mit umfasst.

Das erfindungsgemäße Verfahren kann händisch oder auch automatisiert durchgeführt werden.

Ein Aufstecken eines Konnektors auf das Schlauchende ist nicht erforderlich. Durch die Aufweitung des freien Querschnitts des Schlauchendes ist ein Aufstecken des Schlauches auf die Tülle möglich, ohne dass es zu einer Deformation des Schlauchendes kommt.

Vorzugsweise handelt es sich bei dem Gas um Druckluft, jedoch kommen auch andere Gase in Betracht.

Nach einer optionalen Modifikation der Erfindung kann das Gas, insbesondere die Druckluft, vorgeheizt sein. Darunter ist vorzugsweise zu verstehen, dass das Gas bzw. die Druckluft auf eine Temperatur über Raumtemperatur erwärmt wird. Vorteilhaft hieran ist, dass sich das Schlauchende leichter und/oder stärker aufweitet.

In einer bevorzugten Ausgestaltung der Erfindung ist vorgesehen, dass die Tülle an einer Komponente angeordnet ist, die einen Einlass für das unter Druck stehende Gas aufweist und dass das Verfahren gemäß Schritt a) derart durchgeführt wird, dass das Gas durch den Einlass der Komponente einströmt und durch die Tülle aus der Komponente ausströmt. In diesem Fall strömt die Druckluft oder das sonstige Gas durch den Einlass in die Komponente ein und aus der Tülle wieder aus.

An dieser Stelle wird darauf hingewiesen, dass die Begriffe "ein" und "eine" nicht zwingend auf genau eines der genannten Elemente beschränkt sind, wenngleich dies eine mögliche Ausgestaltung der Erfindung ist. Von den Begriffen sind auch mehrere der fraglichen Elemente umfasst. So kann die Komponente beispielsweise genau einen Einlass oder auch mehrere Einlässe aufweisen.

Vorzugsweise sind an der Komponente mehrere Tüllen angeordnet, wobei die Tüllen, auf die im anstehenden Arbeitsschritt kein Schlauch aufgesteckt werden soll, vorzugsweise luftdicht verschlossen werden. So wird erreicht, dass die Druckluft, die im Folgenden stellvertretend für jedes beliebige Gas verwendet wird, nur oder überwiegend aus der Tülle austritt, auf die ein Schlauch aufgesteckt werden soll.

In einer bevorzugten Ausgestaltung wird zum luftdichten Verschließen eine mit der Tülle form- und/oder kraftschlüssig zusammenwirkende Verschlusskappe verwendet.

Bevorzugt ist es weiterhin, wenn der Schlauch mittels eines Schlauchmontagewerkzeugs gehalten und auf die Tülle aufgesteckt wird. Dabei kann vorgesehen sein, dass die Verringerung des Strömungsquerschnitts gemäß Schritt c) und/oder die Vergrößerung des Strömungsquerschnitts gemäß Schritt e) mittels des Schlauchmontagewerkzeugs vorgenommen werden. Grundsätzlich können für diese Schritte auch andere Mittel verwendet werden.

Besonders vorteilhaft ist es, wenn der Schlauch in Schritt c) derart aufgeweitet wird, dass er ohne Berührung der Außenseite der Tülle auf die Tülle gesteckt wird. Eine Deformation des Schlauchendes wird so besonders zuverlässig verhindert.

Das Verfahren ist insbesondere dann vorteilhaft einsetzbar, wenn die Tülle auf ihrer Außenseite nicht konisch, sondern zylindrisch ausgebildet ist. Konisch zulaufende Tüllen sind hygienisch nachteilig und daher bei Dialysegeräten unüblich. Als zylindrisch werden im gegebenen Zusammenhang sowohl Kreiszylinder als auch Zylinder mit anderen Querschnittsformen (z.B. oval) verstanden. Ferner werden als Zylinder nicht nur solche mit einer glatten Oberfläche, sondern auch solche mit einer gewellten oder geriffelten Oberfläche verstanden.

Um erkennen zu können, dass der Schlauch vollständig aufgesteckt ist, kann vorgesehen sein, dass der Druck an einer geeigneten Stelle gemessen wird, wir z.B. im Schlauch und/oder in der Tülle und/oder in der Komponente und/oder in der Druckluftzufuhr und bei Erreichen eines Druckgrenzwertes auf den aufgesteckten Zustand des Schlauches geschlossen wird. Der Druckanstieg ist darauf zurückzuführen, dass der Ringspalt zwischen dem Schlauchende und der Tülle mit dem zunehmenden Aufstecken kleiner wird.

Das erfindungsgemäße Verfahren wird vorzugsweise an einem Blutbehandlungsgerät und insbesondere an einem Dialysegerät durchgeführt.

Die vorliegende Erfindung betrifft eine Vorrichtung zum Aufstecken eine elastischen Schlauches auf eine formstabile Tülle, wobei die Vorrichtung Folgendes aufweist:
a) Mittel zum Erzeugen einer Strömung von unter Druck stehenden Gas aus der Tülle,
b) Mittel zum Heranführen des Schlauchendes an die Tülle entgegen der Strömungsrichtung des Gases aus der Tülle,
c) Mittel zum Verringern des Strömungsquerschnitts des Schlauchs hinter der Konnektionsstelle zur Erzeugung eines das Schlauchende erweiternden Staudrucks im Schlauch mittels eines Absperrschiebers des Schlauchmontagewerkzeuges, wobei das Schlauchmontagewerkzeug eine elastische und deformierbare Schlauchaufnahme aufweist, die geeignet und bestimmt ist, das Schlauchende aufzunehmen und eine Durchmesservergrößerung des Schlauchendes ermöglicht,
d) Mittel zum Aufstecken des Schlauchendes auf die Tülle und
e) Mittel zum Vergrößern des Strömungsquerschnitts des Schlauchs hinter der Konnektionsstelle zum Anlegen des Schlauchendes an die Außenseite der Tülle aufweist, wobei die Mittel gemäß b) bis e) durch ein und dasselbe Schlauchmontagewerkzeug gebildet werden.

Des Weiteren kann die Vorrichtung Halterungsmittel aufweisen, mittels derer eine Komponente, an der die Tülle angeordnet ist, in einer bestimmten Weise räumlich fixiert wird. Die Komponente, wie z.B. eine Kammer, ein Sensor, ein Aktor, ein hydraulischer Verbinder etc. kann somit definiert im Raum positioniert werden, wodurch die Position der Tülle(n) im Raum festgelegt ist.

Die Mittel gemäß der Merkmale b) bis e) werden durch ein und dasselbe Schlauchmontagewerkzeug gebildet.

In einer möglichen Ausgestaltung der Erfindung weist das Schlauchmontagewerkzeug wenigstens zwei Halbschalen auf, die den Schlauch umgreifen, wobei vorzugsweise vorgesehen ist, dass die Halbschalen aus einem elastischen Material, vorzugsweise aus einem elastischen Kunststoff gebildet sind oder diesen aufweisen. Das elastische Material erlaubt das Aufweiten des Schlauchquerschnitts zum Zwecke des Aufstecken des Schlauches auf die Tülle.

Des Weiteren kann die Vorrichtung Halterungsmittel zum Halten und Bewegen von Verschlusskappen aufweisen, die geeignet und bestimmt sind, Tüllen luftdicht zu verschließen, die nicht mit einem Schlauch verbunden werden sollen.

Weiterhin kann die Vorrichtung mit einem Druckaufnehmer versehen sein, der ausgebildet und angeordnet ist, in der Tülle und/oder in dem Schlauch und/oder in der Druckluftzufuhr und/oder in der Komponente oder an einer sonstigen geeigneten Stelle den Druck zu messen. Der gemessene Druckwert wird an einen Prozessor gemeldet, der ausgebildet ist, den aufgesteckten Zustand des Schlauchendes zu erkennen und/oder zu signalisieren, wenn der gemessene Druckwert einen Grenzwert übersteigt.

Die Vorrichtung kann darüber hinaus mit einer Einrichtung zum Vorheizen der Druckluft versehen sein. Vorteilhaft hieran ist, dass sich dann das Schlauchende leichter und/oder stärker aufweitet.

Nach einer weiteren Fortbildung der Vorrichtung ist der verwendete Schlauch derart ausgestaltet, dass er sich mit erhitzter Luft leichter und/oder stärker aufweiten lässt. Beispielsweise ist dies mit für den Fachmann bekannten Thermoplasten erzielbar.

Die vorliegende Erfindung betrifft schließlich die Verwendung einer Vorrichtung nach einem der Ansprüche 1 bis 5 zum Aufstecken eine elastischen Schlauches auf eine formstabile Tülle eines Blutbehandlungsgerätes, insbesondere eines Dialysegerätes.

Die Tülle kann beispielsweise aus Kunststoff oder aus Metall bestehen.

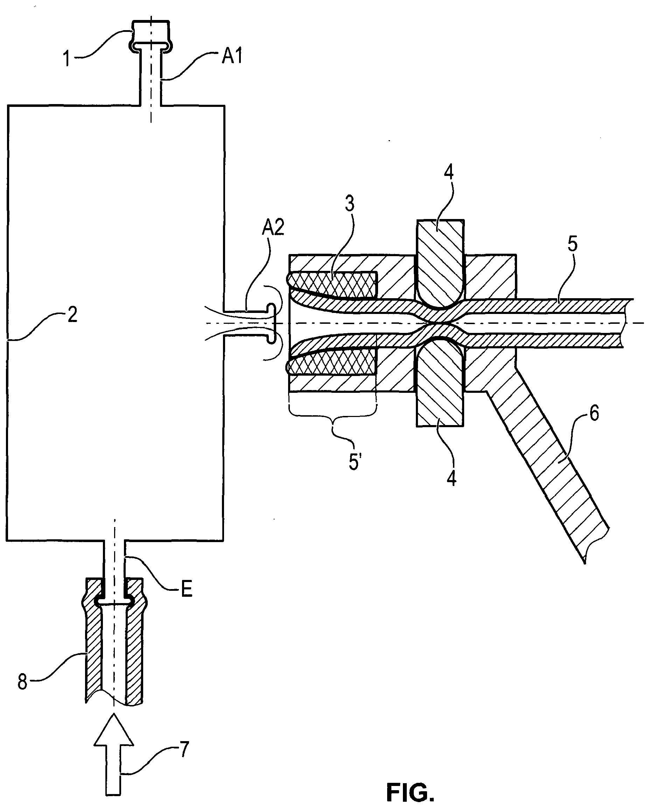

Weitere Einzelheiten und Vorteile der Erfindung werden anhand eines in der Zeichnung dargestellten Ausführungsbeispiels näher erläutert.

Die einzige Figur zeigt eine schematische Schnittdarstellung der beim Aufstecken eines Schlauches beteiligten Komponenten.

Das Bezugszeichen 2 kennzeichnet eine Komponente eines Blutbehandlungsgerätes, wie z. B. eine Kammer, einen Filter, etc.

Die Komponente weist einen Einlass in Form der Tülle E auf, durch den Druckluft 7 durch den Schlauch 8 in die Komponente 2 einströmt. Das Bezugszeichen 7 kennzeichnet eine gesteuerte Druckluftzufuhr mit Druckmessung. Vorzugweise handelt es sich um gereinigte Druckluft.

Die Komponente 2 weist des Weiteren zwei tüllenförmige Auslässe A1 und A2 auf, von denen A1 durch eine Verschlusskappe 1 vollständig oder weitgehend luftdicht verschlossen ist und von denen A2 mit dem Schlauch 5 luftdicht verbunden werden soll. Bei dem Schlauch 5 kann es sich beispielsweise um einen Silikonschlauch handeln.

Das Bezugszeichen 6 kennzeichnet das Schlauchmontagewerkzeug, das den Schlauch fixiert und das zudem einen Absperrschieber 4 aufweist, mittels dessen der Schlauch abgesperrt oder dessen Durchfluss gedrosselt werden kann.

In seinem in der Figur links dargestellten Endbereich weist das Schlauchmontagewerkzeug 6 eine elastische und deformierbare Schlauchaufnahme 3 auf, die das Schlauchende 5' aufnimmt und eine Durchmesservergrößerung des Schlauchendes 5' ermöglicht. Das Schlauchende 5' kann länger als die Tülle A2 ausgeführt sein oder auch dieselbe Länge aufweisen.

Wie dies aus der Figur hervorgeht, sind die Tüllen E, A1 und A2 auf ihrer Außenseite nicht konisch zulaufend ausgeführt, sondern zylindrisch.

Der im Folgenden beschriebene Prozess des Aufsteckens kann manuell oder automatisiert erfolgen.

Zunächst wird die Komponente 2 in eine nicht dargestellte Montagestation eingelegt und somit in einer definierten Lage im Raum positioniert.

Falls die Tüllen E, A1, A2 nicht in einer Fluidverbindung stehen, da die Verbindung durch Ventile getrennt sind, werden diese elektrisch kontaktiert und dadurch geöffnet.

Alle Tüllen (A1) bis auf zwei Tüllen (E, A2) werden luftdicht oder weitgehend luftdicht verschlossen. Dafür wird eine Verschlusskappe 1 verwendet, die dicht um die Tülle A1 abschließt. Die Verschlusskappe 1 besteht aus zwei Halbschalen, die miteinander verrastet werden. Das Aufsetzen der Verschlusskappe 1 kann automatisiert erfolgen, z.B. durch Aufnehmen mittels eines Roboters und Aufsetzen und Verrasten durch ein Montagewerkzeug.

Sodann wird die Komponente 2 an einer Tülle (E) mit Druckluft beaufschlagt, wie dies durch den Pfeil gekennzeichnet ist. Die Druckluft strömt aus der offenen Tülle A2 aus und der Druck der Druckluft wird gemessen, beispielsweise in dem Schlauch 8, in der Komponente 2 etc.

Ein Schlauch 5 wird aus einem Magazin an der Montagestation an einem Ende von dem Schlauchmontagewerkzeug 6 aufgenommen. Das Schlauchmontagewerkzeug 6 drückt den Schlauch 5 hinter der Konnektionsstelle mit der Tülle A2 mittels der Absperrung 4 luftdicht ab.

Sodann wird der Schlauch 5 mit seinem Schlauchende 5' an die Tülle A2 mittels des Schlauchmontagewerkzeugs 6 herangeführt.

Das Schlauchmontagewerkzeug 6 weist zwei Halbschalen auf, die den Schlauch 5 aufnehmen und die aus einem elastischen Kunststoff gefertigt sind. Somit wird der Schlauch 5 einerseits in Position gehalten, andererseits wird eine Aufweitung des Schlauchendes 5' ermöglicht.

Das Schlauchmontagewerkzeug 6 wird weiter an die Tülle A2 herangeführt. Aufgrund der ständig aus dieser Tülle ausströmenden und in das Schlauchende einströmenden Druckluft entsteht ein zunehmender Staudruck, der umso größer wird, je näher der Schlauch an die Tülle 5' heran geführt wird. Der Ringspalt zwischen dem Schlauchende und der Tülle 5' wird kleiner.

Dadurch weitet sich das in dem Bereich 3 befindliche Schlauchende 5' auf. Vorzugsweise erfolgt ein Aufweiten derart, dass der Innendurchmesser des Schlauchendes 5' größer ist als der Außendurchmesser der Tülle A2, so dass das Schlauchende 5' aufgesteckt werden kann, ohne die Tülle A2 zu berühren.

Nun kann der Schlauch mit seinem aufgeweiteten Schlauchende 5' über die Tülle A2 geführt bzw. aufgesteckt werden.

Ist dieser Vorgang abgeschlossen, steigt der Luftdruck in der Komponente 2 oder im Schlauch 8 etc. sprunghaft an. Dies zeigt das Ende des Aufsteckvorgangs an. Am Ende des Aufsteckvorgangs gibt das Schlauchmontagewerkzeug 6 den Schlauch 5 frei und das Absperrelement 4 wird geöffnet. Dadurch sinkt der Staudruck und das Schlauchende 5' legt sich ringsum an die Außenseite der Tülle A2 an.

Soll ein Schlauch an eine weitere Tülle A1 angeschlossen werden, wird deren Verschlusskappe 1 entfernt und der oben beschriebene Vorgang wird wiederholt.

Grundsätzlich ist es auch denkbar, den beschriebenen Konnektionsvorgang an mehreren Tüllen zeitgleich durchzuführen.

Die Tülle E, die zur Luftzufuhr dient, wird am Ende des Aufsteckprozesses ohne Druckluft mit einem Schlauch verbunden.

Das beschriebene Verfahren ist nicht auf eine Komponente beschränkt. Grundsätzlich können auch mehrere Komponenten, wie z.B. Baugruppen auf diese Weise miteinander verbunden werden, da sich die Druckluft über die Schläuche in der Baugruppe verteilt.

Die Druckluft dient nicht nur zur Aufweitung des Schlauchendes, sondern auch als "Gleitmittel", so dass keine Rückstände eines z.B. gelartigen Gleitmittels nach dem Aufstecken entfernt werden müssen.

Über den gemessenen Druck kann die Konnektion zwischen Tülle und Schlauch automatisch auf Dichtheit überwacht werden.

Die oben genannten Schritte können händisch oder automatisiert durchgeführt werden.

So ist beispielsweise der Einsatz eines Roboters mit einem Roboterarm denkbar, der fast jede Position im Raum erreichen kann.

Denkbar ist des Weiteren der Einsatz einer Kamera mit Bilderkennung, so dass der Roboter das Schlauchende exakt positionieren kann.

## Patentansprüche

1. Vorrichtung zum Aufstecken eines elastischen Schlauches auf eine formstabile Tülle (A2), wobei die Vorrichtung
a) Mittel zum Erzeugen einer Strömung von unter Druck stehenden Gas aus der Tülle (A2) und
b) ein Schlauchmontagewerkzeug (6) zum Heranführen des Schlauchendes an die Tülle (A2) entgegen der Strömungsrichtung des Gases aus der Tülle (A2),
c) zum Verringern des Strömungsquerschnitts des Schlauchs (5) hinter der Konnektionsstelle zur Erzeugung eines das Schlauchende (5') erweiternden Staudrucks im Schlauch (5) mittels eines Absperrschiebers (4) des Schlauchmontagewerkzeuges (6),
d) zum Aufstecken des Schlauchendes (5') auf die Tülle (A2) und
e) zum Vergrößern des Strömungsquerschnitts des Schlauchs (5) hinter der Konnektionsstelle zum Anlegen des Schlauchendes (5') an die Außenseite der Tülle (A2) aufweist,
**dadurch gekennzeichnet,**
**dass** das Schlauchmontagewerkzeug (6) eine elastische und deformierbare Schlauchaufnahme (3) aufweist, die geeignet und bestimmt ist, das Schlauchende (5') aufzunehmen und eine Durchmesservergrößerung des Schlauchendes (5') ermöglicht.

2. Vorrichtung nach Anspruch 1, **dadurch gekennzeichnet, dass** die Vorrichtung Halterungsmittel aufweist, mittels derer eine Komponente, an der die Tülle (A2) angeordnet ist, in einer bestimmten Weise räumlich fixiert wird und/oder dass die Vorrichtung Mittel zum Erwärmen des Gases aufweist.

3. Vorrichtung nach Anspruch 2, **dadurch gekennzeichnet, dass** das Schlauchmontagewerkzeug (6) wenigstens zwei Halbschalen aufweist, die den Schlauch (5) umgreifen, wobei vorzugsweise vorgesehen ist, dass die Halbschalen aus einem elastischen Material, vorzugsweis aus einem elastischen Kunststoff gebildet sind oder diesen aufweisen.

4. Vorrichtung nach einem der Ansprüche 1 bis 3, **dadurch gekennzeichnet, dass** die Vorrichtung Halterungsmittel zum Halten und Bewegen von Verschlusskappen (1) aufweist, die geeignet und bestimmt sind, Tüllen (A1) luftdicht zu verschließen, die nicht mit einem Schlauch verbunden werden sollen.

5. Vorrichtung nach einem der Ansprüche 1 bis 4, **dadurch gekennzeichnet, dass** ein Druckaufnehmer vorgesehen ist, der ausgebildet und angeordnet ist, in der Tülle (A2), in dem Schlauch (5), in der Druckluftzufuhr (7) oder in der Komponente (2) den Druck zu messen und dass die Vorrichtung einen Prozessor aufweist, dem der gemessene Druckwert zugeführt wird und dass der Prozessor ausgebildet ist, den aufgesteckten Zustand des Schlauchendes (5') zu erkennen und/oder zu signalisieren, wenn der gemessene Druckwert einen Grenzwert übersteigt.

6. Verfahren zum Aufstecken eines elastischen Schlauches (5) auf eine formstabile Tülle (A2) mittels einer Vorrichtung nach einem der vorhergehenden Ansprüche, wobei das Verfahren die folgenden Schritte umfasst:
a) Erzeugen einer Strömung von unter Druck stehendem Gas aus der Tülle (A2),
b) Heranführen des Schlauchendes (5') an die Tülle (A2) entgegen der Strömungsrichtung des Gases aus der Tülle (A2)
c) Verringern des Strömungsquerschnitts des Schlauchs (5) hinter der Konnektionsstelle zur Erzeugung eines das Schlauchende (5') erweiternden Staudrucks im Schlauch (5) mittels eines Schlauchmontagewerkzeuges (6), mittels dessen der Schlauch (5) abgesperrt oder dessen Durchfluss gedrosselt wird,
d) Aufstecken des Schlauchendes (5') auf die Tülle (A2) und
e) Vergrößern des Strömungsquerschnitts des Schlauchs (5) hinter der Konnektionsstelle zum Anlegen des Schlauchendes (5') an die Außenseite der Tülle (A2), wobeider Schlauch (5) mittels des Schlauchmontagewerk-zeugs (6) gehalten und auf die Tülle (A2) aufgesteckt wird und vorgesehen ist, dass die Verringerung des Strömungsquerschnitts gemäß Schritt c) sowie die Vergrößerung des Strömungsquerschnitts gemäß Schritt e) mittels des Schlauchmontagewerkzeugs (6) vorgenommen werden, wobei die Vergrößerung des Strömungsquerschnitts gemäß Schritt e) durch Freigeben des Schlauches (5) aus dem Schlauchmontagewerkzeug (6) erfolgt.

7. Verfahren nach Anspruch 6, **dadurch gekennzeichnet, dass** es sich bei dem Gas um Druckluft handelt und/oder dass das Gas erwärmt ist.

8. Verfahren nach Anspruch 6 oder 7, **dadurch gekennzeichnet, dass** die Verfahrensschritte b) bis e) manuell oder automatisiert durchgeführt werden.

9. Verfahren nach einem der Ansprüche 6 bis 8, **dadurch gekennzeichnet, dass** die Tülle (A2) an einer Komponente (2) angeordnet ist, die einen Einlass (E) für das unter Druck stehende Gas aufweist und dass das Verfahren gemäß Schritt a) derart durchgeführt wird, dass das Gas durch den Einlass (E) der Komponente (2) einströmt und durch die Tülle (A2) aus der Komponente (2) ausströmt.

10. Verfahren nach Anspruch 9, **dadurch gekennzeichnet, dass** an der Komponente (2) mehrere Tüllen (A1, A2) angeordnet sind und dass die Tüllen (A1), auf die kein Schlauch aufgesteckt werden soll, luftdicht verschlossen werden, wobei zum luftdichten Verschließen vorzugsweise eine mit der Tülle (A1) form- und/oder kraftschlüssig zusammenwirkende Verschlusskappe (1) verwendet wird.

11. Verfahren nach einem Ansprüche 6 bis 10, **dadurch gekennzeichnet, dass** der Schlauch (5) in Schritt c) derart aufgeweitet wird, so dass er ohne Berührung der Außenseite der Tülle (A2) auf die Tülle (A2) gesteckt wird.

12. Verfahren nach einem der Ansprüche 6 bis 11, **dadurch gekennzeichnet, dass** die Tülle (A2) auf ihrer Außenseite nicht konisch, sondern zylindrisch ausgebildet ist.

13. Verfahren nach einem der Ansprüche 6 bis 12, **dadurch gekennzeichnet, dass** der Druck im Schlauch (5) und/oder in der Tülle (A2) und/oder in der Komponente (2) und/oder in der Druckluftzufuhr (7) gemessen wird und bei Erreichen eines Druckgrenzwertes auf den aufgesteckten Zustand des Schlauches (5) geschlossen wird.

14. Verfahren nach einem der Ansprüche 6 bis 13, **dadurch gekennzeichnet, dass** sich die Tülle (A2) an einem Blutbehandlungsgerät und insbesondere an einem Dialysegerät befindet.

15. Verwendung einer Vorrichtung nach einem der Ansprüche 1 bis 5 zum Aufstecken eine elastischen Schlauches (5) auf eine formstabile Tülle (A2) eines Blutbehandlungsgerätes, insbesondere eines Dialysegerätes.

## Claims

1. Apparatus for attaching an elastic hose onto a dimensionally stable spout (A2), wherein the device comprises:
a) means for generating a flow of pressurized gas from the spout (A2); and
b) a hose assembly tool (6) for guiding the hose end toward the spout (A2) against the direction of flow of the gas from the spout (A2);
c) for reducing the flow cross-section of the hose (5) downstream of the connection point for generating a dynamic pressure in the hose (5) that expands the hose end (5') by means of a gate valve (4) of the hose assembly tool (6);
d) for attaching the hose end (5') to the spout (A2); and
e) for increasing the flow cross-section of the hose (5) downstream of the connection point for applying the hose end (5') to the outer side of the spout (A2),
**characterized in that**
the hose assembly tool (6) comprises an elastic and deformable hose receptacle (3), which is suitable and intended for receiving the hose end (5`) and allows for an increase in diameter of the hose end (5').

2. Apparatus in accordance with claim 1, **characterized in that** the apparatus has holding means by means of which a component at which the spout (A2) is arranged is spatially fixed in a specific manner and/or that the apparatus comprises means for heating the gas.

3. Apparatus in accordance with claim 2, **characterized in that** the hose assembly tool (6) has at least two half-shells that engage around the hose (5), wherein provision preferably is made that the half-shells are formed from or comprise an elastic material, preferably an elastic plastic.

4. Apparatus in accordance with one of the claims 1 to 3, **characterized in that** the apparatus comprises holding means for holding and moving closure caps (1) that are suited and intended to close spouts (A1) that are not to be connected to a hose in an airtight manner.

5. Apparatus in accordance with one of the claims 1 to 4, **characterized in that** a pressure sensor is provided that is configured and arranged to measure the pressure in the spout (A2), in the hose (5), in the compressed air supply (7) or in the component (2); and **in that** the apparatus comprises a processor to which the measured pressure value is supplied; and **in that** the processor is configured to recognize the attached state of the hose end (5') and/or to signal when the measured pressure value exceeds a limit value.

6. Method for attaching an elastic hose (5) to a dimensionally stable spout (A2) according to an apparatus according to one of the preceding claims, wherein the method comprises the following steps:
a) generating a flow of pressurized gas from the spout (A2);
b) guiding the hose end (5') toward the spout (A2) against the direction of flow of the gas from the spout (A2);
c) reducing the flow cross-section of the hose (5) after the connection point for generating a dynamic pressure that expands the hose end (5') in the hose (5) by means of a hose assembly tool (6), by means of which the hose (5) is shutoff or the flow therethrough is choked,
d) attaching the hose end (5') to the spout (A2); and
e) increasing the flow cross-section of the hose (5) after the connection point for applying the hose end (5') to the outer side of the spout (A2), wherein the hose (5) is held by means of a hose assembly tool (6) and is attached to the spout (A2), wherein provision is made that the reduction of the flow cross-section in accordance with step c) and the increase of the flow cross-section in accordance with step e) are carried out by means of the hose assembly tool (6), wherein increasing the flow cross-section according to step e) is effected by releasing the hose (5) from the hose assembly tool (6).

7. Method in accordance with claim 6, **characterized in that** the gas is compressed air and/or that the gas is heated.

8. Method in accordance with claim 6 or claim 7, **characterized in that** the method steps b) to e) are carried out manually or in an automated manner.

9. Method in accordance with one of claims 6 to 8, **characterized in that** the spout (A2) is arranged at a component (2) that comprises an inlet (E) for the pressurized gas; and **in that** the method in accordance with step a) is carried out such that the gas flows in through the inlet (E) of the component (2) and flows out of the component (2) through the spout (2).

10. Method in accordance with claim 9, **characterized in that** a plurality of spouts (A1, A2) are arranged at the component (2); and **in that** the spouts (A1) onto which no hose is to be attached are closed in an airtight manner, wherein a closure cap (1) is preferably used for the airtight closing that cooperates with the spout (A1) in a shape-matching and/or force-transmitting manner.

11. Method in accordance with one of claims 6 to 10, **characterized in that** the hose (5) is widened in step c) such that it is pushed onto the spout (A2) without contacting the outer side of the spout (A2).

12. Method in accordance with one of claims 6 to 11, **characterized in that** the spout (A2) is not conical, but rather cylindrical at its outer side.

13. Method in accordance with one claims 6 to 12, **characterized in that** the pressure in the hose (5) and/or in the spout (A2) and/or in the component (2) and/or in the compressed air supply (7) is measured; and **in that** an attached state of the hose (5) is concluded when a limit pressure value is reached.

14. Method in accordance with one of claims 6 to 13, **characterized in that** the spout (A2) is located at a blood treatment device and in particular at a dialysis machine.

15. Use of an apparatus in accordance with one of the claims 1 to 5 for attaching an elastic hose (5) to a dimensionally stable spout (A2) of a blood treatment device, in particular of a dialysis machine.

## Revendications

1. Dispositif pour emmancher un flexible élastique sur une douille de raccord (A2) indéformable, le dispositif présentant
a) des moyens destinés à générer un écoulement de gaz sous pression hors de la douille de raccord (A2) et
b) un outil de montage de flexible (6) destiné à amener l'extrémité du flexible à la douille de raccord (A2) dans le sens contraire à l'écoulement du gaz hors de la douille de raccord (A2),
c) réduire la section de passage du flexible (5) derrière le point de connexion pour générer, dans le flexible (5), une pression de retenue élargissant l'extrémité du flexible (5') au moyen d'une vanne d'arrêt (4) de l'outil de montage de flexible (6),
d) emmancher l'extrémité du flexible (5') sur la douille de raccord (A2) et
e) augmenter la section de passage du flexible (5) derrière le point de connexion pour appliquer l'extrémité du flexible (5') sur le côté extérieur de la douille de raccord (A2),
**caractérisé en ce que**
l'outil de montage de flexible (6) présente un logement de flexible (3) élastique et déformable, qui est approprié et destiné à la réception de l'extrémité du flexible (5') et qui permet une augmentation du diamètre de l'extrémité du flexible (5').

2. Dispositif selon la revendication 1, **caractérisé en ce que** le dispositif présente des moyens de retenue au moyen desquels un composant sur lequel est disposée la douille de raccord (A2) est fixé dans l'espace d'une manière définie et/ou **en ce que** le dispositif présente des moyens destinés à chauffer le gaz.

3. Dispositif selon la revendication 2, **caractérisé en ce que** l'outil de montage de flexible (6) présente au moins deux demi-coques qui enveloppent le flexible (5), les demi-coques étant de préférence prévues pour être formées par un matériau élastique, de préférence une matière plastique élastique ou pour en contenir.

4. Dispositif selon l'une des revendications 1 à 3, **caractérisé en ce que** le dispositif présente des moyens de retenue destinés à retenir et déplacer des capuchons (1) qui sont appropriés et destinés à la fermeture de manière étanche à l'air de douilles de raccord (A1) qui ne doivent pas être reliées à un flexible.

5. Dispositif selon l'une des revendications 1 à 4, **caractérisé en ce qu'**un capteur de pression est prévu, lequel est conçu et agencé pour mesurer la pression dans la douille de raccord (A2), dans le flexible (5), dans l'arrivée d'air comprimé (7) ou dans le composant (2) et **en ce que** le dispositif présente un processeur auquel la valeur de pression mesurée est transmise et **en ce que** le processeur est conçu pour reconnaître et/ou signaler l'état emmanché de l'extrémité du flexible (5') quand la valeur de pression mesurée dépasse une valeur limite.

6. Procédé pour emmancher un flexible (5) élastique sur une douille de raccord (A2) indéformable au moyen d'un dispositif selon l'une des revendications précédentes, dans lequel le procédé comprend les étapes suivantes consistant à :
a) générer un écoulement de gaz sous pression hors de la douille de raccord (A2),
b) amener l'extrémité du flexible (5') à la douille de raccord (A2) dans le sens contraire à l'écoulement du gaz hors de la douille de raccord (A2)
c) réduire la section de passage du flexible (5) derrière le point de connexion pour générer, dans le flexible (5), une pression de retenue élargissant l'extrémité du flexible (5') au moyen d'un outil de montage de flexible (6), au moyen duquel le flexible (5) peut être fermé ou son débit étranglé,
d) emmancher l'extrémité du flexible (5') sur la douille de raccord (A2) et
e) augmenter la section de passage du flexible (5) derrière le point de connexion pour appliquer l'extrémité du flexible (5') sur le côté extérieur de la douille de raccord (A2), le flexible (5) étant maintenu et emmanché sur la douille de raccord (A2) au moyen de l'outil de montage de flexible (6) et la diminution de la section de passage selon l'étape c) ainsi que l'augmentation de la section de passage selon l'étape e) étant prévues pour être effectuées au moyen de l'outil de montage de flexible (6), l'augmentation de la section de passage selon l'étape e) étant effectuée en libérant le flexible (5) de l'outil de montage de flexible (6).

7. Procédé selon la revendication 6, **caractérisé en ce que** le gaz est de l'air comprimé et/ou **en ce que** le gaz est chauffé.

8. Procédé selon la revendication 6 ou 7, **caractérisé en ce que** les étapes de procédé b) à e) sont exécutées manuellement ou de manière automatisée.

9. Procédé selon l'une des revendications 6 à 8, **caractérisé en ce que** la douille de raccord (A2) est agencée sur un composant (2) qui présente une entrée (E) pour le gaz sous pression et **en ce que** le procédé selon l'étape a) est exécuté de telle manière que le gaz afflue par l'entrée (E) du composant (2) et s'échappe hors du composant (2) par la douille de raccord (A2).

10. Procédé selon la revendication 9, **caractérisé en ce que** plusieurs douilles de raccord (A1, A2) sont agencées sur le composant (2) et **en ce que** les douilles de raccord (A1) sur lesquelles aucun flexible n'est à emmancher sont fermées de manière étanche à l'air, un capuchon (1) qui coopère par complémentarité de forme et/ou par adhérence avec la douille de raccord (A1) étant de préférence utilisé pour la fermeture étanche à l'air.

11. Procédé selon l'une des revendications 6 à 10, **caractérisé en ce que**, dans l'étape c), le flexible (5) est élargi de manière à être emmanché sur la douille de raccord (A2) sans toucher le côté extérieur de la douille de raccord (A2).

12. Dispositif selon l'une des revendications 6 à 11, **caractérisé en ce que** la douille de raccord (A2) est réalisée non pas conique, mais cylindrique sur son côté extérieur.

13. Procédé selon l'une des revendications 6 à 12, **caractérisé en ce que** la pression dans le flexible (5) et/ou dans la douille de raccord (A2) et/ou dans le composant (2) et/ou dans l'arrivée d'air comprimé (7) est mesurée et lorsque une valeur limite de pression est atteinte, il est conclu à l'état emmanché du flexible (5).

14. Procédé selon l'une des revendications 6 à 13, **caractérisé en ce que** la douille de raccord (A2) se trouve sur un appareil de traitement du sang et en particulier sur un appareil de dialyse.

15. Utilisation d'un dispositif selon l'une des revendications 1 à 5, pour emmancher un flexible (5) élastique sur une douille de raccord (A2) indéformable d'un appareil de traitement du sang, en particulier d'un appareil de dialyse.
